# EUROPEAN PATENT APPLICATION

(11) **EP 3 842 045 A1**
(43) Date of publication of application: **30.06.2021**
(21) Application number: 19852841.6
(22) Date of filing: 14.08.2019
(51) Int. Cl.: A61K 31/702, A61K 31/192, A61K 31/194, A61K 31/60, A61K 31/7004, A61K 31/715, A61K 35/74, A61K 36/06, A61P 35/00, C12P 7/40

(54) **MEDICINAL PRODUCT, ANTICANCER AGENT, MEDICINAL INTERMEDIATE PRODUCT, AND METHOD FOR PRODUCING CYCLIC CARBOXYLIC ACID COMPOUND OR DERIVATIVE THEREOF**

(30) Priority: 23.08.2018 JP 2018156531
(71) Applicant: Sumitomo Bakelite Co.Ltd., Shinagawa-ku Tokyo 140-0002 (JP); Research Institute Of Innovative Technology For The Earth, Kizugawa-shi, Kyoto 619-0292 (JP)
(72) Inventor: INOUE, Yusuke, Tokyo 140-0002 (JP); FUJIWARA, Daisuke, Tokyo 140-0002 (JP); TACHIBANA, Kenya, Tokyo 140-0002 (JP); MIYAUCHI, Hiroyuki, Tokyo 140-0002 (JP)
(74) Representative: Müller-Boré & Partner Patentanwälte PartG mbB
(86) International application number: PCT/JP2019/031962
(87) International publication number: WO 2020/040017

(57) **Abstract**

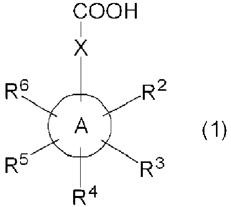

The medicinal product of the present invention contains, as an active ingredient, at least one of a cyclic carboxylic acid compound derived from a plant-derived saccharide and a microorganism, and a derivative thereof. Further, it is preferable that the cyclic carboxylic acid compound is a compound represented by Formula (1).

## Description

### Technical Field

The present invention relates to a medicinal product, an anticancer agent, a medicinal intermediate product, and a method for producing a cyclic carboxylic acid compound or a derivative thereof.

### Background Art

Aromatic compounds having specific structures are known to be used as one of components of medicinal products. For example, PTL 1 describes that aromatic hydroxycarboxylic acid is important as a raw material or an intermediate product of a medicinal product.

### Citation List

### Patent Literature

[PTL 1] JP-A-2007-238469

### Summary of Invention

### Technical Problem

However, aromatic compounds such as those mentioned above are usually obtained from petroleum. In such a case, a petroleum-derived aromatic compound (chemical products) which can be recovered by fractional purification of petroleum is a so-called fundamental compound having a simple chemical structure. On the other hand, a high value-added compound having a more complicated chemical structure should be necessarily derived from the fundamental compound through a synthesis process. In this case, various isomers derived from raw materials and synthesis processes, trace components derived from catalysts, ionic components, mineral components, and the like can remain in a petroleum-derived chemical product unless the petroleum is thoroughly fractionated and refined regardless of production cost. It cannot be said that such impurities included in the petroleum-derived chemical product are preferable from the viewpoint of safety to human bodies.

An object of the present invention is to provide a medicinal product and an anticancer agent, each of which contains, as an active ingredient, at least one of a cyclic carboxylic acid compound and a derivative thereof, includes no petroleum-derived impurities, and is thus safer; and a medicinal intermediate product which is an intermediate product of the medicinal product or the anticancer agent. Another object of the present invention is to provide a method for producing a cyclic carboxylic acid compound or a derivative thereof, which can be used as an active ingredient of a medicinal product or an anticancer agent.

### Solution to Problem

Such objects are achieved by the present invention as described in the following (1) to (11).
(1) A medicinal product containing, as an active ingredient, at least one of a cyclic carboxylic acid compound derived from a plant-derived saccharide and a microorganism, and a derivative of the cyclic carboxylic acid compound.
(2) The medicinal product as described in (1),
   in which the cyclic carboxylic acid compound is a compound represented by Formula (1), 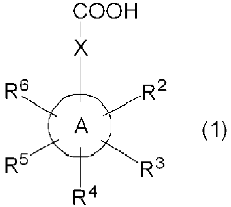 where a ring A is a 5-membered ring of a saturated ring, a partially saturated ring, or an aromatic ring, or a 6-membered ring of a saturated ring, a partially saturated ring, or an aromatic ring, X is a single bond or a bond including one or more carbon atoms, and R² to R⁶ (R² to R⁵ in a case where the ring A is the 5-membered ring) are each independently a hydrogen atom, a hydroxyl group, an amino group, an alkoxy group, a carboxyl group, or a carbonyl group.
(3) The medicinal product as described in (2),
   in which the cyclic carboxylic acid compound is at least one selected from the group consisting of 2-hydroxybenzoic acid, 3-hydroxybenzoic acid, 4-hydroxybenzoic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, and 3,5-dihydroxybenzoic acid.
(4) The medicinal product as described in (2),
   in which in a case where the ring A of the cyclic carboxylic acid compound is the 5-membered ring of the saturated ring or partially saturated ring whose all ring-constituting atoms are carbon atoms, one or more of the carbon atoms of the ring A to which R² to R⁵ and X are bonded are asymmetric carbon atoms, and in a case where the ring A of the cyclic carboxylic acid compound is the 6-membered ring of the saturated ring or partially saturated ring whose all ring-constituting atoms are carbon atoms, one or more of the carbon atoms of the ring A to which R² to R⁶ and X are bonded are asymmetric carbon atoms.
(5) The medicinal product as described in (4),
   in which in the cyclic carboxylic acid compound, in a case where a carbon atom of the ring A to which X is bonded is defined as C¹, a carbon atom of the ring A to which R² is bonded is defined as C², a carbon atom of the ring A to which R³ is bonded is defined as C³, a carbon atom of the ring A to which R⁴ is bonded is defined as C⁴, a carbon atom of the ring A to which R⁵ is bonded is defined as C⁵, and a carbon atom of the ring A to which R⁶ is bonded is defined as C⁶, a combination of carbon atoms which are asymmetric carbon atoms is one selected from the group consisting of the following (a) to (h),
   (a) C¹,
   (b) C²,
   (c) C³,
   (d) C⁴,
   (e) C¹ and C⁴,
   (f) C³ and C⁴,
   (g) C¹, C³, and C⁴, and
   (h) C³, C⁴, and C⁵.
(6) The medicinal product as described in (1),
   in which the cyclic carboxylic acid compound or the derivative thereof is 3-dehydroquinate, 3-dehydroshikimic acid, shikimic acid, chorismic acid, or prephenic acid.
(7) The medicinal product as described in any one of (1) to (6),
   in which the microorganism is Escherichia coli, Bacillus subtilis, Staphylococcus aureus, Corynebacterium glutamicum, actinomycetes, cyanobacteria, Methanobacterium thermoautotrophicum, Halobacterium salinarum, Alicyclobacillus acidoterrestris, acid-fast bacterium, a fungus, a yeast, or a transformant thereof.
(8) The medicinal product according to any one of (1) to (7),
   in which the raw material of the plant-derived saccharide is an inedible biomass resource.
(9) An anticancer agent containing, as an active ingredient, at least one of a cyclic carboxylic acid compound derived from a plant-derived saccharide and a microorganism, and a derivative of the cyclic carboxylic acid compound.
(10) A medicinal intermediate product containing at least one of a cyclic carboxylic acid compound derived from a plant-derived saccharide and a microorganism, and a derivative of the cyclic carboxylic acid compound.
(11) A method for producing a cyclic carboxylic acid compound or a derivative thereof, which is used as an active ingredient of a medicinal product or an anticancer agent, including:
   a step of preparing a culture solution including a plant-derived saccharide and a microorganism to produce at least one of the cyclic carboxylic acid compound and the derivative thereof;
   a step of concentrating the culture solution to obtain a concentrated solution; and
   a step of collecting at least one of the cyclic carboxylic acid compound and the derivative thereof from the concentrated solution by a crystallization method, a precipitation method, an extraction method, a sublimation purification method, or a distillation method.

### Advantageous Effects of Invention

According to the present invention, it is possible to provide a medicinal product and anticancer agent, each of which contains, as an active ingredient, at least one of a cyclic carboxylic acid compound and a derivative thereof, and includes no petroleum-derived impurities; and a medicinal intermediate product containing at least one of a cyclic carboxylic acid compound and a derivative thereof. It is therefore possible to efficiently produce a medicinal product, an anticancer agent, and a medicinal intermediate product, which are safer than a medicinal product, an anticancer agent, and a medicinal intermediate product, each containing petroleum-derived impurities, respectively.

### Description of Embodiments

Hereinafter, a medicinal product, an anticancer agent, a medicinal intermediate product, and a method for producing a cyclic carboxylic acid compound or a derivative thereof of the present invention will be described in detail, based on preferred embodiments.

### <<Medicinal Product>>

The present inventors have conducted intensive studies, and as a result, they have found that it is possible to provide a medicinal product including no petroleum-derived impurities by using, as an active ingredient, at least one of a cyclic carboxylic acid compound derived from plant-derived saccharides and a microorganism, and a derivative thereof. At this time, the present inventors have found that it is preferable to produce the cyclic carboxylic acid compound or a derivative thereof by a bioprocess using plant-derived saccharides (raw material) and a microorganism.

That is, the medicinal product of the present invention contains, as an active ingredient, at least one of a cyclic carboxylic acid compound derived from plant-derived saccharides and a microorganism, and a derivative thereof. In other words, the medicinal product of the present invention contains, as an active ingredient, at least one of a cyclic carboxylic acid compound produced by a reaction between plant-derived saccharides and a microorganism (bioprocess), and a derivative thereof.

As a result, it is possible to provide a medicinal product including no petroleum-derived impurities. Such a medicinal product is safer than a medicinal product including petroleum-derived impurities.

In addition, at least one of a cyclic carboxylic acid compound derived from plant-derived saccharides and a microorganism, and a derivative thereof is also used as a raw material (medicinal intermediate product) which enables the production of a medicinal product.

As a result, it is possible to provide a medicinal raw material which contains, as an active ingredient, at least one of a cyclic carboxylic acid compound and a derivative thereof, and includes no petroleum-derived impurities. Such a medicinal intermediate product enables the production of a medicinal product which is safer than a medicinal intermediate product containing petroleum-derived impurities.

The cyclic carboxylic acid compound contained in the medicinal product or a raw material thereof is not particularly limited, but is preferably a compound represented by Formula (1) . 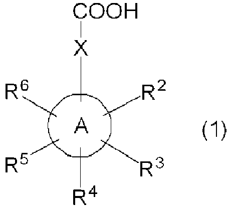 [in Formula (1), the ring A is a 5-membered ring of a saturated ring, a partially saturated ring, or an aromatic ring, or a 6-membered ring of a saturated ring, a partially saturated ring, or an aromatic ring, X is a single bond or a bond including one or more carbon atoms, and R² to R⁶ (R² to R⁵ in a case where the ring A is a 5-membered ring) are each independently a hydrogen atom, a hydroxyl group, an amino group, an alkoxy group, a carboxyl group, or a carbonyl group].

Examples of the 5-membered ring of a saturated ring, a partially saturated ring, or an aromatic ring include a furan structure, a thiophene structure, a pyrrole structure, a pyrrolidine structure, a tetrahydrofuran structure, a 2,3-dihydrofuran structure, a pyrazole structure, an imidazole structure, an oxazole structure, an isoxazole structure, a thiazole structure, and an isothiazole structure.

Examples of the 6-membered ring of a saturated ring include hydrocarbon-based saturated rings such as a cyclohexane structure, nitrogen-containing saturated rings such as a piperidine structure, a piperazine structure, a triazinane structure, a tetrazinane structure, a pentazinane structure, and a quinuclidine structure, oxygen-containing saturated rings such as a tetrahydropyran structure and a morpholine structure, and sulfur-containing saturated rings such as a tetrahydrothiopyran structure.

Examples of the 6-membered ring of a partially saturated ring includes hydrocarbon-based partially saturated rings such as a cyclohexene structure and a cyclohexadiene structure, nitrogen-containing partially saturated rings such as a piperidine structure, oxygen-containing partially saturated rings such as a pyran structure, and sulfur-containing partially saturated rings such as a thiazine structure.

Examples of the 6-membered ring of an aromatic ring includes hydrocarbon-based aromatic rings such as a benzene structure, and nitrogen-containing aromatic rings (nitrogen-containing unsaturated rings) such as a pyridine structure, a pyridazine structure, a pyrimidine structure, a pyrazine structure, a triazine structure, a tetrazine structure, and a pentazine structure.

X is a single bond or a bond including one or more carbon atoms.

In a case where X is the single bond, a carboxyl group is directly bonded to a ring-constituting atom of the ring A.

On the other hand, examples of the bond including one or more carbon atoms include a hydrocarbon group having 1 to 4 carbon atoms, an ether bond, an ester bond, an amide bond, a carbonyl group, and a vinylidene group, which can be used singly or in combination of two or more kinds thereof.

Among those, the hydrocarbon group having 1 to 4 carbon atoms may be linear or branched, and may be saturated or unsaturated. Further, a hydrogen atom of the hydrocarbon group may be substituted with a substituent such as an alkyl group having 1 or 2 carbon atoms, a hydroxyl group, an amino group, a carboxyl group, or a halogen atom.

In a case where the Ring A is the 6-membered ring, R² to R⁶ are each independently a hydrogen atom, a hydroxyl group, an amino group, an alkoxy group, a carboxyl group, or a carbonyl group. In a case where the ring A is the 5-membered ring, R² to R⁵ are each independently a hydrogen atom, a hydroxyl group, an amino group, an alkoxy group, a carboxyl group, or a carbonyl group.

Moreover, in a case where any of R² to R⁶ in the ring A which is the 6-membered ring, or any of R² to R⁵ in the ring A which is the 5-membered ring is a carbonyl group, a structure in which the ring-constituting atom of the ring A is a carbon atom, and the carbon atom and the oxygen atom are linked via a double bond is referred to as a carbonyl group.

Specific examples of the cyclic carboxylic acid compound represented by Formula (1) include benzoic acid, phthalic acid, isophthalic acid, terephthalic acid, hemimellitic acid, trimellitic acid, trimesic acid, mellophanic acid, prehnitic acid, pyromellitic acid, phenylacetic acid, hydroxyphenylacetic acid, phenylbutyric acid (phenyl lactate), hydroxyphenylbutyric acid, phenylpyruvic acid, hydroxyphenylpyruvic acid, phenyllactic acid, hydroxyphenyllactic acid, anthranilic acid, hydroatropic acid, atropic acid, hydrocinnamic acid (coumaric acid), silicic acid, salicylic acid (2-hydroxybenzoic acid), m-salicylic acid (3-hydroxybenzoic acid), p-salicylic acid (4-hydroxybenzoic acid), methoxybenzoic acid, aminobenzoic acid, hydroxybenzoic acid, pyrocatechuic acid (2,3-dihydroxybenzoic acid), β-resorcylic acid (2,4-dihydroxybenzoic acid), gentisic acid (2,5-dihydroxybenzoic acid), γ-resorcylic acid (2,6-dihydroxybenzoic acid), protocatechuic acid (3,4-dihydroxybenzoic acid), α-resorcylic acid (3,5-dihydroxybenzoic acid), trihydroxybenzoic acid, vanillic acid (4-hydroxy-3-methoxybenzoic acid), isovanillic acid (3-hydroxy-4-methoxybenzoic acid), veratric acid, gallic acid, syringic acid, asaronic acid, mandelic acid, vanillylmandelic acid, anisic acid, homogentisic acid, homoprotocatechuic acid, homovanillic acid, homoisovanillic acid, homoveratric acid, homophthalic acid, homoisophthalic acid, homoterephthalic acid, phthalonic acid, isophthalonic acid, terephthalonic acid, atrolactic acid, tropic acid, melilotoic acid, phloretic acid, dihydrocaffeic acid, hydroferulic acid, hydroisoferulic acid, umbellic acid, caffeic acid (coffee acid), ferulic acid, isoferulic acid, sinapic acid, syringic acid, dehydroquinic acid, dehydroshikimic acid, shikimic acid, chorismic acid, L-tryptophane, L-tyrosine, prephenic acid, arogenic acid, and L-phenylalanine.

Examples of the derivative of the cyclic carboxylic acid compound include an ester, an acid anhydride, an amide, an acid halide, a salt, and the like of the above-mentioned compounds, or all compounds derived from the cyclic carboxylic acid compound.

Among those, the cyclic carboxylic acid compound represented by Formula (1) is particularly preferably at least one selected from the group consisting of 2-hydroxybenzoic acid, 3-hydroxybenzoic acid, 4-hydroxybenzoic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, and 3,5-dihydroxybenzoic acid. By using these, it is possible to realize a medicinal product which has various types of efficacy and is safer.

The derivative of the cyclic carboxylic acid compound represented by Formula (1) is preferably erlotinib, itopride hydrochloride, veratridine, hydrochloric acid Mai Pi skin Lin, or Picatin II. By using these, it is possible to realize a medicinal product having more excellent efficacy and being safer.

Moreover, among those derivatives, any of erlotinib, itopride hydrochloride, and veratridine are compounds derived from the cyclic carboxylic acid compound represented by Formula (1), and structures thereof are represented by the following formulae.

### • Erlotinib

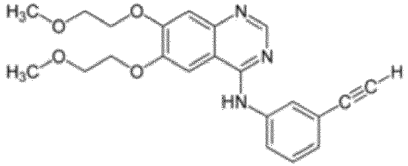

### • Itopride hydrochloride

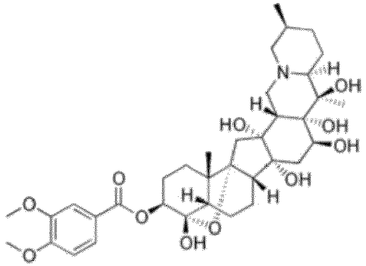

### • Veratridine

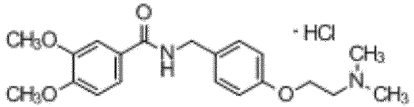

On the other hand, hydrochloric acid Mai Pi skin Lin and Picatin II are compounds derived from the cyclic carboxylic acid compound represented by Formula (1), and are described as examples of a medicinal product in the following documents.

### Global Protocatechuic Acid (CAS 99-50-3) Market Research Report 2018

The molecular weight of the cyclic carboxylic acid compound or a derivative thereof is not particularly limited, but is preferably 120 to 1,000, and more preferably 130 to 800.

Furthermore, in a case where the ring A of the cyclic carboxylic acid compound represented by Formula (1) is a 5-membered ring of a saturated ring or partially saturated ring where all of the ring-constituting atoms are carbon atoms, it is preferable that one or more of the carbon atoms of the ring A to which R² to R⁵ and X are bonded are asymmetric carbon atoms. In addition, in a case where the ring A of the cyclic carboxylic acid compound represented by Formula (1) is a 6-membered ring of a saturated ring or partially saturated ring where all ring-constituting atoms are carbon atoms, it is preferable that one or more of the carbon atoms of the ring A to which R² to R⁶ and X are bonded are asymmetric carbon atoms.

In such a case, the cyclic carboxylic acid compound becomes a stereoisomer, which makes it possible to realize a significant medicinal product or a raw material which enables the production of such a medicinal product. Further, by producing such a cyclic carboxylic acid compound from plant-derived saccharides, a medicinal product including a specific stereoisomer with high purity or a raw material thereof can be obtained. That is, it is possible to obtain a medicinal product or a raw material thereof, including a specific stereoisomer with high purity and having a low content of other stereoisomers. Such a medicinal product or a raw material thereof is useful from the viewpoint of realizing a medicinal product having excellent safety and efficacy. In addition, since a complicated production process accompanying the removal of unnecessary stereoisomers is not required, the production cost can be lowered.

Moreover, in the cyclic carboxylic acid compound represented by Formula (1), in a case where a carbon atom of the ring A to which X is bonded is defined as C¹, a carbon atom of the ring A to which R² is bonded is defined as C², a carbon atom of the ring A to which R³ is bonded is defined as C³, a carbon atom of the ring A to which R⁴ is bonded is defined as C⁴, a carbon atom of the ring A to which R⁵ is bonded is defined as C⁵, and a carbon atom of the ring A to which R⁶ is bonded is defined as C⁶, it is preferable that a combination in which carbon atoms are asymmetric carbon atoms is one selected from the group consisting of the following (a) to (h).
(a) C¹,
(b) C²,
(c) C³,
(d) C⁴,
(e) C¹ and C⁴,
(f) C³ and C⁴,
(g) C¹, C³, and C⁴, and
(h) C³, C⁴, and C⁵.

Incidentally, Formula (2) is a formula in which the indications of C¹ to C⁶ are added to the cyclic carboxylic acid compound represented by Formula (1).

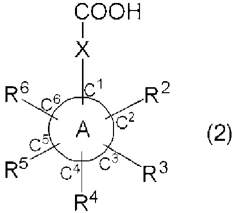

[In Formula (2), the ring A is a 5-membered ring of a saturated ring, a partially saturated ring, or an aromatic ring, or a 6-membered ring of a saturated ring, a partially saturated ring, or an aromatic ring, X is a single bond or a bond including one or more carbon atoms, and R² to R⁶ (R² to R⁵ in a case where the ring A is a 5-membered ring) are each independently a hydrogen atom, a hydroxyl group, an amino group, an alkoxy group, a carboxyl group, or a carbonyl group. Further, C¹ to C⁶ are each a carbon atom as a ring-constituting atom of the ring A.]

In such a case, the cyclic carboxylic acid compound becomes a stereoisomer, which makes it possible to realize a particularly significant medicinal product or a raw material which enables the production of such a medicinal product. Further, by producing such a cyclic carboxylic acid compound from plant-derived saccharides, a medicinal product including a specific stereoisomer which is one selected from the group consisting of (a) to (h) above with high purity or a raw material thereof can be obtained. That is, it is possible to obtain a medicinal product or a raw material thereof, including a specific stereoisomer with high purity and having a low content of other stereoisomers. Such a medicinal product or a raw material thereof is useful from the viewpoint of realizing a medicinal product having excellent safety and efficacy. In addition, since a complicated production process accompanying the removal of unnecessary stereoisomers can be reduced, the production cost can be lowered.

Moreover, the cyclic carboxylic acid compound and a derivative thereof according to the present invention are compounds represented by Formula (2), and in particular, 3-dehydroquinate, 3-dehydroshikimic acid, shikimic acid, chorismic acid, or prephenic acid is preferable. All of these compounds can be produced from plant-derived saccharides, and are useful as an active ingredient of a medicinal product or a medicinal raw material (medicinal intermediate product). Therefore, by using these compounds produced from plant-derived saccharides, it is possible to realize a medicinal product which has more excellent efficacy and is safer.

In addition, the structures of these cyclic carboxylic acid compounds are represented by the following formulae.

### • 3-Dehydroquinate

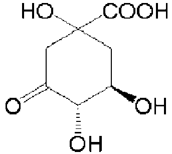

### • 3-Dehydroshikimic acid

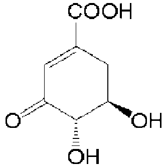

### • Shikimic acid

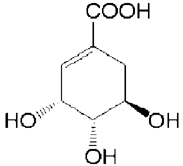

### • Chorismic acid

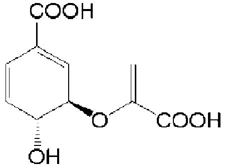

### • Prephenic acid

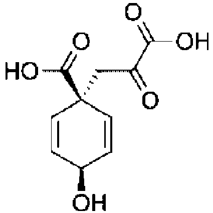

Furthermore, in addition to those above, examples of the cyclic carboxylic acid compound or a derivative thereof according to the present invention include compounds represented by Formulae (a) to (y).

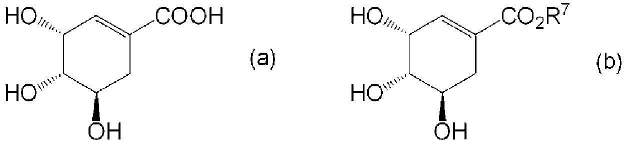

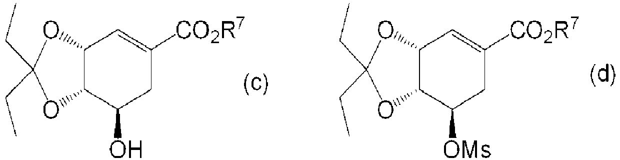

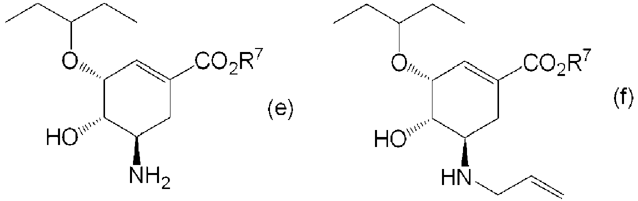

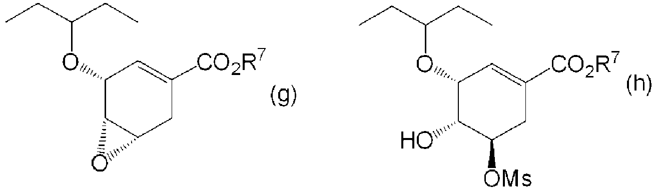

[In Formulae (a) to (h), R⁷ represents a linear or branched alkyl group having 1 to 12 carbon atoms, and Ms represents a mesyl group.]

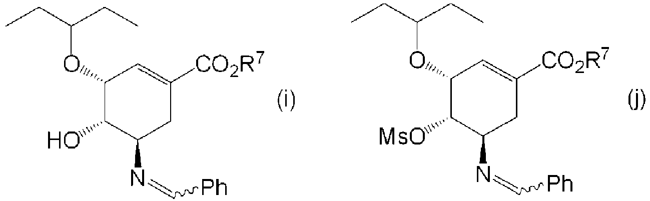

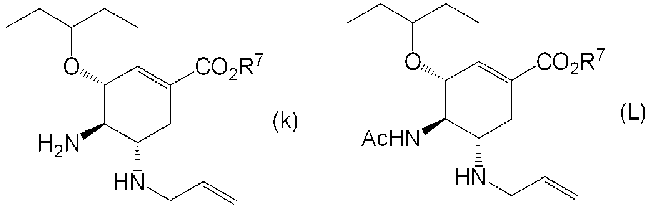

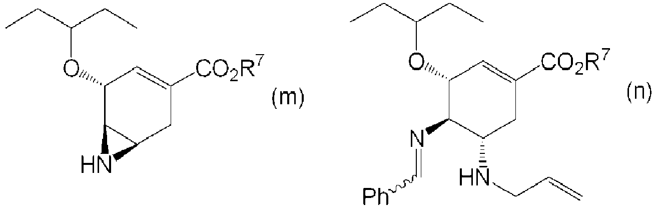

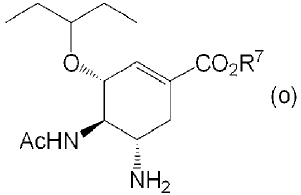

[In Formulae (i) to (o), R⁷ represents a linear or branched alkyl group having 1 to 12 carbon atoms, Ms represents a mesyl group, Ph represents a phenyl group, and Ac represents an acrylic group.]

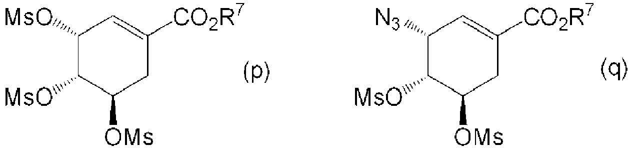

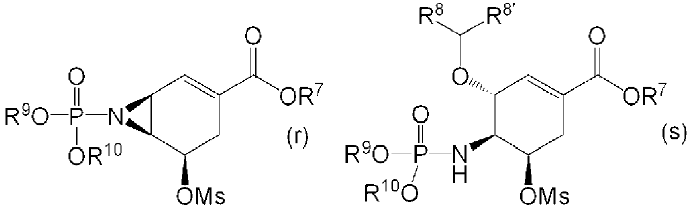

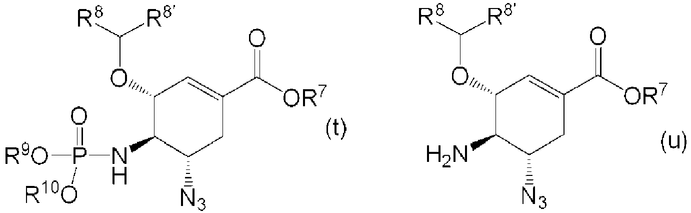

[In Formulae (p) to (u), R⁷ represents a linear or branched alkyl group having 1 to 12 carbon atoms, R⁸ and R^{8'} each independently represent a linear or branched alkyl group having 1 to 12 carbon atoms, R⁹ and R¹⁰ each independently represent a linear or branched alkyl group having 1 to 12 carbon atoms, and Ms represents a mesyl group.]

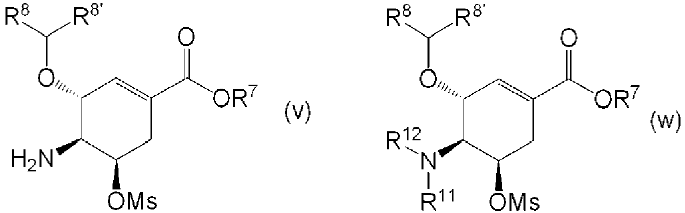

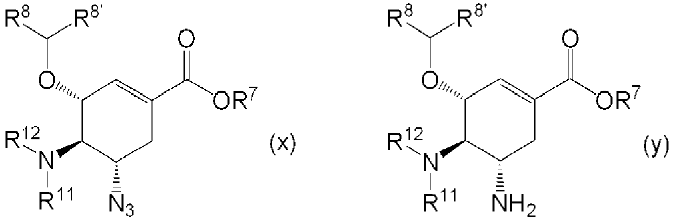

[In Formulae (v) to (y), R⁷ represents a linear or branched alkyl group having 1 to 12 carbon atoms, R⁸ and R^{8'} each independently represent a linear or branched alkyl group having 1 to 12 carbon atoms, R¹¹ and R¹² each independently represent a hydrogen atom or an alkanoyl group, and Ms represents a mesyl group.]

Examples of the alkanoyl group include a hexanoyl group, a pentanoyl group, a butanoyl group, a propanoyl group, an ethanoyl group (acetyl group), and a methanoyl group.

Furthermore, the compound represented by Formula (o) among Formulae (a) to (y) is oseltamivir. Oseltamivir phosphate is useful as an active ingredient of a therapeutic agent for influenza.

Therefore, by producing such a compound from plant-derived saccharides, it is possible to provide a therapeutic agent for influenza, which includes no petroleum-derived impurities and is safer, at low cost.

Examples of the medicinal product containing, as an active ingredient, at least one of such the cyclic carboxylic acid compound and a derivative thereof include, in addition to the therapeutic agent for influenza, an anticancer agent, an antiulcer agent, an antidiabetic agent, an antifibrotic agent, an antiaging agent, an antivirus agent, an antiinflammatory agent, an analgesic, an antiarteriosclerosis agent, an antilipid agent, a cardiac stimulant, a liver protecting agent, a neuroprotective agent, a kidney protecting agent, an antireproductive toxicity agent, an antiasthma agent, and an antispasmodic agent.

Here, the anticancer agents of the present invention each contain, as an active ingredient, at least one of a cyclic carboxylic acid compound derived from plant-derived saccharides and a microorganism, and a derivative thereof. In other words, the anticancer agents of the present invention each contain, as an active ingredient, at least one of a cyclic carboxylic acid compound produced by a reaction between plant-derived saccharides and a microorganism (bioprocess), and a derivative thereof.

As a result, it is possible to provide an anticancer agent including no petroleum-derived impurities. Such an anticancer agent is safer than an anticancer agent including petroleum-derived impurities.

On the other hand, examples of the medicinal raw material (medicinal intermediate product) containing at least one of the cyclic carboxylic acid compound and a derivative thereof include an intermediate product for producing the above-mentioned medicinal product.

Furthermore, at least one of such a cyclic carboxylic acid compound and a derivative thereof can be applied to uses other than the medicinal product and the medicinal raw material (medicinal intermediate product). Examples of such uses include health supplement compositions, food additives, flavors, food products, cosmetics, daily necessities, cleaning agents, and quasi-drugs.

Among these, examples of the food product include oral compositions (gums, candies, and the like), fish paste products such as Japanese kamaboko and chikuwa, animal products such as sausages and hams, western confectioneries, Japanese confectioneries, noodles such as Chinese noodles, Japanese udon noodles, and Japanese soba noodles, seasonings such as sauce, soysauce, and baste, side dishes, juices, and soups.

In addition, examples of the cosmetics include skin lotions, milky lotions, creams, foundations, eye shadows, lipsticks, cheek rouges, hair cosmetics, emollient creams, emollient lotions, creams, cream rinses, cold creams, vanishing creams, lotions, pack agents, gels, face packs, soaps, body soaps, shampoos, conditioners, hair rinses, bathing agents, bath medicines, pigments, shaving creams, hair creams, hair lotions, hair treatments, hair packs, glosses, and lip creams.

### <<Method for Producing Cyclic Carboxylic Acid Compound or Derivative thereof>>

The above-mentioned cyclic carboxylic acid compound or a derivative thereof is produced by a bioprocess using a microorganism with plant-derived saccharides as a raw material. That is, the above-mentioned cyclic carboxylic acid compound or a derivative thereof is derived from plant-derived saccharides and a microorganism.

The plant-derived saccharides are not particularly limited, and examples thereof include a monosaccharide, a polysaccharide, and a mixture thereof.

The monosaccharide is not particularly limited, and examples thereof include a saccharide which can be treated with a transformant of a microorganism which will be described later. Examples of such a saccharide (monosaccharides) include tetrose (C4 saccharide), pentose (C5 saccharide), hexose (C6 saccharide), and heptose (C7 saccharide) from the viewpoint of improving the phenol productivity of a transformant. Among these, as the monosaccharide, at least one selected from the group consisting of arabinose, xylose, glucose, mannitol, fructose, mannose, galactose, and sucrose is particularly preferable. In addition, such saccharides may be used singly or in combination of two or more kinds thereof as a mixed saccharide.

The polysaccharide is a polymer of monosaccharides. The average degree of polymerization of the polysaccharide is not particularly limited, but is preferably 2 or more and 100 or less, and more preferably 2 or more and 50 or less, from the viewpoint of improving the productivity in a bioprocess using a microorganism. Further, the polysaccharides may be used singly or in combination of two or more kinds thereof. Examples of the polysaccharide include maltose, lactose, cellobiose, xylobiose, trehalose, acarbose, stachyose, fructooligosaccharide, galactooligosaccharide, and mannanoligosaccharide.

The plant-derived saccharides are preferably produced from an inedible biomass resource. In other words, it is preferable that the raw material of the plant-derived saccharides is an inedible biomass resource.

As the biomass resource, various biomass resources can be used as long as they include at least a monosaccharide or a polysaccharide from the viewpoint of obtaining the above-mentioned saccharides. Examples of the biomass resource include vegetation resources typified by weeds generated from urban areas or cultivated land, thinned wood in forest production areas, and the like, as well as waste celluloses, waste starches, or waste molasses recovered as process residues or wastes in the general food industry, sugar cane pomace in the sugar industry, and sake lees and shochu lees in the brewing industry, which can be used singly or in combination of two or more kinds thereof. Further, a processed product can also be used as the biomass resource.

The plant-derived saccharides can be obtained by saccharifying such a biomass resource. As such plant-derived saccharides, cellulose-derived saccharides obtained by saccharifying waste cellulose are preferable, and a cellulose-derived mixed saccharide is more preferable. Hereinafter, a process using a microorganism with the plant-derived saccharides as a raw material will be described in detail.

The process using a microorganism with the plant-derived saccharides as a raw material is a process having a step of obtaining a culture solution including at least one of the cyclic carboxylic acid compound produced by a microorganism using the plant-derived saccharides as a raw material (produced from the conversion of the plant-derived saccharides by the microorganism) and a derivative thereof, a step of concentrating the culture solution to obtain a concentrated solution, and a step of recovering at least one of the cyclic carboxylic acid compound and a derivative thereof from the concentrated solution by a crystallization method, a precipitation method, an extraction method, a sublimation purification method, or a distillation method. By performing such a process, at least one of the cyclic carboxylic acid compound and a derivative thereof can be efficiently obtained. In addition, according to the process using such a microorganism, the active ingredient is produced (generated) as a result of a reaction between the raw material and the microorganism. Therefore, by limiting the types of the raw material and the microorganism (for example, limiting the raw material to plant-derived saccharides and limiting the type of the microorganism to bacteria), only one active ingredient (a cyclic carboxylic acid compound or a derivative thereof) can be obtained efficiently. Such an effect can be remarkably exhibited by further limiting the type of the raw material and the microorganism (for example, limiting the raw material to a cellulose-derived mixed saccharide and limiting the type of bacteria). From this point, the process is different from a process which does not use a microorganism (for example, a process which uses an extract from a plant). From this viewpoint, the cyclic carboxylic acid compound derived from plant-derived saccharides and a microorganism, and a derivative thereof can be said to be a cyclic carboxylic acid compound and a derivative thereof (excluding those derived from a plant extract).

In the bioprocess, the recovery rate of the cyclic carboxylic acid compound and a derivative thereof can be improved by appropriately selecting a microorganism, a medium, a culture equipment, and culture conditions.

### <Step of Preparing Culture Solution>

First, a culture solution including a raw material, a microorganism, a medium, and the like is prepared. At least one of a cyclic carboxylic acid compound and a derivative thereof is produced by culturing the microorganism in the culture solution, and reacting the raw material and the like with the microorganism (by a bioprocess).

The type of the microorganism is not particularly limited as long as it can produce the cyclic carboxylic acid compound and a derivative thereof with high efficiency. Generally, the microorganism is selected from bacteria such as Escherichia coli, Bacillus subtilis, Staphylococcus aureus, and Corynebacterium glutamicum, actinomycetes such as Streptomyces griseus, cyanobacteria such as Microcystis aeruginosa, archaebacteria such as Methanobacterium thermoautotrophicum, Halobacterium salinarum, Sulfolobus acidocaldarius, Alicyclobacillus acidoterrestris, and acid-fast bacterium, fungi such as Aspergillus oryzae, and yeasts such as Saccharomyces cerevisiae, depending on purposes, and a transformant thereof obtained by a known method as needed is utilized.

As the medium, a medium usually used for culturing a microorganism may be applied. The medium includes medium components in order to prepare an environment necessary for the growth of a microorganism. The medium component is preferably contained in an appropriate amount depending on the type of the microorganism using a carbon source, a nitrogen source, inorganic salts, other nutrients, and the like. Therefore, the culture solutionbefore the bioprocess is preferably formed of raw materials, microorganisms, and medium components.

Examples of the carbon source include sugars or sugar alcohols such as glucose, fructose, sucrose, mannose, maltose, mannitol, xylose, arabinose, galactose, starch, sugar honey, sorbitol, and glycerin; organic acids such as acetic acid, citric acid, lactic acid, fumaric acid, maleic acid, and gluconic acid; alcohols such as ethanol and propanol. As the carbon source, one of these may be used singly, or two or more thereof may be mixed and used.

Examples of the nitrogen source include inorganic or organic ammonium compounds such as ammonium chloride, ammonium sulfate, ammonium nitrate, and ammonium acetate, urea, aqueous ammonia, sodium nitrate, and potassium nitrate. In addition, a nitrogen-containing organic compound such as a corn steep liquor, a meat extract, peptone, NZ-amine, a protein hydrolyzate, and an amino acid can also be used. As the nitrogen source, one of these may be used singly, or two or more kinds thereof may be mixed and used.

Examples of the inorganic salts include primary potassium phosphate, secondary potassium phosphate, magnesium sulfate, sodium chloride, ferrous nitrate, manganese sulfate, zinc sulfate, cobalt sulfate, and calcium carbonate. As the inorganic salts, one of these may be used singly, or two or more kinds thereof may be mixed and used.

Examples of the nutrient include a meat extract, peptone, polypeptone, a yeast extract, a dried yeast, a corn steep liquor, a skim milk powder, a skim soybean hydrochloric acid hydrolyzate, an animal, plant, or microbial cell extract, and a decomposition product thereof. In addition, vitamins can also be added to the medium as needed. Examples of the vitamins include biotin, thiamine (vitamin B1), pyridoxine (vitamin B6), pantothenic acid, inositol, and nicotinic acid.

The culture equipment may be of any of a batch type, a fed-batch type, and a continuous type, but the batch type is preferable in a case where high-product production is supposed. Moreover, in general, a seed culture method is often adopted, which starts from a flask-scale culture and is stepwise expanded to cell expansion culture, and a group of incubators whose sizes are different in several stages depending on the production scale are used as a set. Furthermore, with regard to the culture conditions, the medium temperature is preferably about 15°C to 45°C, and the pH of the medium is preferably about 6 to 8. In addition, parameters such as a ventilation method and a ventilation amount to a culture tank, a stirring method, a rotation speed, the shape of a stirring blade, and a culture time are appropriately set depending on the scale and the specifications of a culture equipment, and the type and the concentration of microorganisms used, and the culture process is appropriately adjusted by real-time monitoring.

### <Concentrating Step, and Isolating and Purifying Step>

A culture solution obtained by a bioprocess can be prepared in a state where the cyclic carboxylic acid compound or a derivative thereof is contained at a suitable concentration in a case where a microbial growth environment is appropriately selected. For the purpose of selectively and efficiently recovering the cyclic carboxylic acid compound or a derivative thereof from the culture solution prepared in this manner, a recovering process including a culture solution concentrating step and an isolating and purifying step is applied. According to this method, a cyclic carboxylic acid compound or a derivative thereof can be efficiently produced.

The concentrating step improves the concentration of at least one of the cyclic carboxylic acid compound and a derivative thereof contained in the culture solution obtained after the bioprocess, and the subsequent isolating and purifying step is performed for the purpose of recovering a target compound with a high yield and a high purity. Hereinafter, the concentrating step will be described.

The culture solution after the bioprocess includes, in addition to at least one of the cyclic carboxylic acid compound and a derivative thereof produced by the bioprocess, a carbon source, a nitrogen source, inorganic salts, nutrients, and the like as medium components, and also contains organic acids, amino acids, and salts thereof which are by-produced during the bioprocess. In addition, 70% to 99% of the total weight of the culture solution after the bioprocess is usually water. Therefore, in the concentrating step, it is desirable to efficiently remove moisture without deteriorating or depleting the cyclic carboxylic acid compound or a derivative thereof and also without further increasing the amount of by-products produced in association with the concentration. To achieve this purpose, chemical engineering techniques such as heating concentration, vacuum distillation, solvent extraction, solid extraction, membrane separation, or the like can be applied, but the vacuum concentration is particularly suitably used in order to avoid deterioration and depletion of the cyclic carboxylic acid compound or a derivative thereof by heat or oxidation during the concentrating step, and to reduce the amount of heat energy input associated with water removal.

The isolating and purifying step is performed for the purpose of selectively recovering at least one of the cyclic carboxylic acid compound and a derivative thereof from the concentrated solution obtained by the concentrating step.

In the isolating and purifying step, various chemical engineering methods such as water vapor distillation, precision fractional distillation, temperature crystallization, acid crystallization, salting out, reprecipitation, sublimation, column purification, extraction, and membrane separation can be applied. A suitable method is selected in consideration of the properties of a target compound and the properties of impurities and by-products to be removed. The properties of the cyclic carboxylic acid compound are different depending on the type and number of a substituent, but the cyclic carboxylic acid compound or a derivative thereof is solid at room temperature, and in a case where the water solubility of impurities and by-products is relatively high, a crystallization method (temperature crystallization or acid crystallization) is suitably used.

### <Processing into Medicinal Product>

The medicinal product of the present invention may include any other components within a scope where the effects of the present invention are not impaired.

Examples of other components include sugar, condensed milk, wheat flour, shortening, salts, glucose, chicken eggs, butters, margarines, candies, calcium, iron, seasonings, spices, and oil components (an animal or plant oil, a mineral oil, an ester oil, a wax oil, a silicone oil, a higher alcohol, a phospholipids, fatty acids, and the like), surfactants (an anionic, cationic, amphoteric, or nonionic surfactants), vitamins (a vitamin A group, a vitamin B group, folic acid, nicotinic acid, pantothenic acid, biotin, a vitamin C group, a vitamin D group, a vitamin E group, other ferulic acids, γ-oryzanol, and the like), UV absorbers (p-aminobenzoic acid, anthranyl acid, salicylic acid, coumarin, benzotriazole, tetrazole, imidazoline, pyrimidine, dioxane, furan, pyrone, camphor, nucleic acid, allantoin or a derivative thereof, an amino acid compound, Shikonin, baicalin, baicalein, berberine, and the like), antioxidants (a stearic ester, nordihydroguaceretenic acid, dibutylhydroxytoluene, butylhydroxyanisole, parahydroxyanisole, propyl gallate, sesamol, sesamolin, gossypol, and the like), thickeners (hydroxyethyl cellulose, ethyl cellulose, carboxyethyl cellulose, methyl cellulose, carboxymethyl cellulose, sodium carboxymethyl cellulose, hydroxypropyl cellulose, nitrocellulose, polyvinyl alcohol, polyvinyl methyl ether, polyvinylpyrrolidone, polyvinyl methacrylate, polyacrylate, carboxyvinyl polymer, arabic rubber, tragacanth rubber, agar, casein, dextrin, gelatin, pectin, starch, alginic acid or a salt thereof, and the like), moisturizers (propylene glycol, 1,3-butylene glycol, polyethylene glycol, glycerin, 1,2-pentanediol, hexylene glycol, octylene glycol, chondroitin sulfate or a salt thereof, hyaluronic acid or a salt thereof, sodium lactate, and the like), lower alcohols, polyhydric alcohols, water-soluble polymers, pH adjusters, antiseptic or antifungal agents, colorants, fragrances, refreshing agents, stabilizers, animal or plant extracts, animal or plant proteins or decomposition products thereof, animal or plant polysaccharides or decomposition products thereof, animal or plant glycoproteins or decomposition products thereof, microbial culture metabolic components, blood flow promoters, antiinflammatory agents, antiinflammatory agents, antiallergic agents, cell activators, amino acids or salts thereof, keratolytic agents, astringents, wound healing agents, defoaming agents, oral agents, deodorants, and emulsifiers. Incidentally, it should be noted that these can be used singly or in combination of two or more kinds thereof.

Moreover, the medicinal product of the present invention may be in any of forms, but it is, for example, in a solution form, a cream form, a paste form, a gelled form, a gel form, a foam form, a solid form, a powder form, or the like.

In addition, depending on the purpose, use, and usage of the medicinal product of the present invention, it is also possible to apply spraying or transpiration to a method of using the medicinal product of the present invention.

Although the medicinal product or a raw material (medicinal intermediate product) thereof of the present invention has been described above, specific examples of the medicinal product are not limited to those described above and may be any of the products.

### Examples

Hereinafter, the present invention will be described more specifically, based on Examples.

### (Example 1)

### <Production of 3,4-Dihydroxybenzoic Acid by Bioprocess>

A culture solution obtained by a bioprocess using plant-derived saccharides and an microorganism was concentrated under reduced pressure so that the total concentration of solid contents was 15% to 30% by mass. The decompression degree was 100 to 5,000 Pa, the liquid temperature was 30°C to 80°C, and a concentration treatment was performed with vacuum distillation equipment. Although the concentration degree depends on the treatment time, a concentrated solution having a total concentration of solid contents of 15% to 30% by mass was recovered by the concentration treatment for 6 to 8 hours. Hydrochloric acid was added to this concentrated solution to adjust the pH to 4 or less, and the mixture was further cooled to 0°C to room temperature. A crystallized product was recovered by filtration, appropriately washed, and then dried under reduced pressure to recover high-purity 3,4-dihydroxybenzoic acid having a purity of 99% or more.

Furthermore, this high-purity 3,4-dihydroxybenzoic acid, that is, the cyclic carboxylic acid compound included no petroleum-derived impurities.

### (Example 2)

### <Production of Shikimic Acid by Bioprocess>

Activated carbon was added to a culture solution obtained by a bioprocess using plant-derived saccharides and a microorganism, and a treatment with activated carbon was performed. Next, a column filled with an ion exchange resin was prepared and treated with a 2 mol/L aqueous sodium hydroxide solution. The ion exchange resin used is a strongly basic anion exchange resin. Pure water was passed through the column until the effluent became neutral, the raw material liquid treated with activated carbon was then passed through the column, and subsequently, pure water was passed. Thereafter, a 2 mol/L aqueous acetic acid solution was passed as an eluent to recover the acidic fraction. The concentration of shikimic acid was measured for each of the recovered fractions, and the eluent was passed through until the elution of shikimic acid was completed. The eluent was subjected to concentrated crystallization to precipitate a sold, thereby obtaining solid shikimic acid. Incidentally, the concentration crystallization is a process in which a concentrated treatment and a cooling crystallization treatment are sequentially performed to precipitate solid shikimic acid.

Furthermore, this high-purity shikimic acid, that is, the cyclic carboxylic acid compound included no petroleum-derived impurities.

### Industrial Applicability

According to the present invention, it is possible to provide a medicinal product and anticancer agent, each of which contains, as an active ingredient, at least one of a cyclic carboxylic acid compound and a derivative thereof, and includes no petroleum-derived impurities; and a medicinal intermediate product containing at least one of a cyclic carboxylic acid compound and a derivative thereof. It is therefore possible to efficiently produce a medicinal product, an anticancer agent, and a medicinal intermediate product, which are safer than a medicinal product, an anticancer agent, and a medicinal intermediate product, each containing petroleum-derived impurities, respectively. Therefore, the present invention has industrial applicability.

## Claims

1. A medicinal product comprising, as an active ingredient, at least one of a cyclic carboxylic acid compound derived from a plant-derived saccharide and a microorganism, and a derivative of the cyclic carboxylic acid compound.

2. The medicinal product according to claim 1,
wherein the cyclic carboxylic acid compound is a compound represented by Formula (1),
where a ring A is a 5-membered ring of a saturated ring, a partially saturated ring, or an aromatic ring, or a 6-membered ring of a saturated ring, a partially saturated ring, or an aromatic ring, X is a single bond or a bond including one or more carbon atoms, and R² to R⁶ (R² to R⁵ in a case where the ring A is the 5-membered ring) are each independently a hydrogen atom, a hydroxyl group, an amino group, an alkoxy group, a carboxyl group, or a carbonyl group.

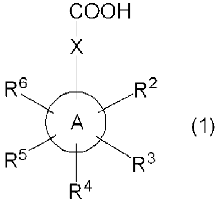

3. The medicinal product according to claim 2,
wherein the cyclic carboxylic acid compound is at least one selected from the group consisting of 2-hydroxybenzoic acid, 3-hydroxybenzoic acid, 4-hydroxybenzoic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, and 3,5-dihydroxybenzoic acid.

4. The medicinal product according to claim 2,
wherein in a case where the ring A of the cyclic carboxylic acid compound is the 5-membered ring of the saturated ring or partially saturated ring whose all ring-constituting atoms are carbon atoms, one or more of the carbon atoms of the ring A to which R² to R⁵ and X are bonded are asymmetric carbon atoms, and in a case where the ring A of the cyclic carboxylic acid compound is the 6-membered ring of the saturated ring or partially saturated ring whose all ring-constituting atoms are carbon atoms, one or more of the carbon atoms of the ring A to which R² to R⁶ and X are bonded are asymmetric carbon atoms.

5. The medicinal product according to claim 4,
wherein in the cyclic carboxylic acid compound, in a case where a carbon atom of the ring A to which X is bonded is defined as C¹, a carbon atom of the ring A to which R² is bonded is defined as C², a carbon atom of the ring A to which R³ is bonded is defined as C³, a carbon atom of the ring A to which R⁴ is bonded is defined as C⁴, a carbon atom of the ring A to which R⁵ is bonded is defined as C⁵, and a carbon atom of the ring A to which R⁶ is bonded is defined as C⁶, a combination of carbon atoms which are asymmetric carbon atoms is one selected from the group consisting of the following (a) to (h) ;
(a) C¹,
(b) C²,
(c) C³,
(d) C⁴,
(e) C¹ and C⁴,
(f) C³ and C⁴,
(g) C¹, C³, and C⁴, and
(h) C³, C⁴, and C⁵.

6. The medicinal product according to claim 1,
wherein the cyclic carboxylic acid compound or the derivative thereof is 3-dehydroquinate, 3-dehydroshikimic acid, shikimic acid, chorismic acid, or prephenic acid.

7. The medicinal product according to any one of claims 1 to 6,
wherein the microorganism is Escherichia coli, Bacillus subtilis, Staphylococcus aureus, Corynebacterium glutamicum, actinomycetes, cyanobacteria, Methanobacterium thermoautotrophicum, Halobacterium salinarum, Alicyclobacillus acidoterrestris, acid-fast bacterium, a fungus, a yeast, or a transformant thereof.

8. The medicinal product according to any one of claims 1 to 7,
wherein a raw material of the plant-derived saccharide is an inedible biomass resource.

9. An anticancer agent comprising, as an active ingredient, at least one of a cyclic carboxylic acid compound derived from a plant-derived saccharide and a microorganism, and a derivative of the cyclic carboxylic acid compound.

10. A medicinal intermediate product comprising at least one of a cyclic carboxylic acid compound derived from a plant-derived saccharide and a microorganism, and a derivative of the cyclic carboxylic acid compound.

11. A method for producing a cyclic carboxylic acid compound or a derivative thereof, which is used as an active ingredient of a medicinal product or an anticancer agent, comprising:
a step of preparing a culture solution including a plant-derived saccharide and a microorganism to produce at least one of the cyclic carboxylic acid compound and the derivative thereof;
a step of concentrating the culture solution to obtain a concentrated solution; and
a step of collecting at least one of the cyclic carboxylic acid compound and the derivative thereof from the concentrated solution by a crystallization method, a precipitation method, an extraction method, a sublimation purification method, or a distillation method.
